(19) Europäisches Patentamt
European Patent Office
Office européen des brevets

(11) Numéro de publication : **0 652 214 A1**

# (12) DEMANDE DE BREVET EUROPEEN

(21) Numéro de dépôt : **94402478.5**

(22) Date de dépôt : **03.11.94**

(51) Int. Cl.$^{6}$ : **C07D 307/91,** C07D 307/79, C07C 255/23, A61K 31/275, A61K 31/34

(30) Priorité : **04.11.93 GB 9322781**

(43) Date de publication de la demande :
**10.05.95 Bulletin 95/19**

(84) Etats contractants désignés :
**AT BE CH DE DK ES FR GB GR IE IT LI LU NL PT SE**

(71) Demandeur : **ROUSSEL-UCLAF**
**35, Boulevard des Invalides**
**F-75007 Paris (FR)**

(72) Inventeur : **Evans, Phillip L.**
**Hoechst Roussel,**
**Kingfisher Drive**
**Covingham, Swindon, Wiltshire SN3 5BZ (GB)**
Inventeur : **Kuo, Elizabeth Anne**
**8, Bullfinch Close**
**Covingham, Swindon, Wiltshire SN3 5PH (GB)**

(74) Mandataire : **Vieillefosse, Jean-Claude et al**
**ROUSSEL UCLAF,**
**111 Route de Noisy**
**F-93235 Romainville Cédex (FR)**

(54) **Nouveaux dérivés N-aryl 2-cyano 3-hydroxy propénamides, leur procédé de préparation, leur application comme médicaments et les compositions pharmaceutiques les renfermant.**

(57) Les composés de formule générale (I) :

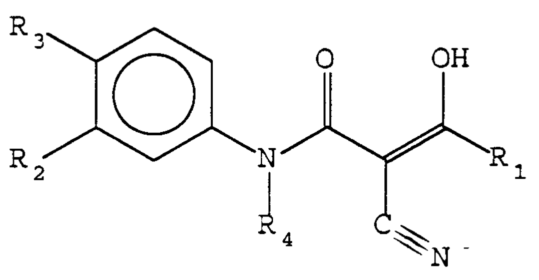

(I)

[dans laquelle
$R_1$ représente un groupe alkyle contenant de 1 à 6 atomes de carbone, un groupe cycloalkyle contenant de 3 à 6 atomes de carbone, un groupe alcényle contenant de 2 à 6 atomes de carbone ou un groupe alcynyle contenant de 2 à 6 atomes de carbone ;
$R_2$ et $R_3$, avec les atomes qui les portent, forment un système à noyau carbocyclique ou hétérocyclique, éventuellement substitué ; et
$R_4$ représente un atome d'hydrogène ou un groupe alkyle contenant de 1 à 3 atomes de carbone] ; leurs tautomères ; et leurs sels d'addition avec les bases possèdent une activité anti-inflammatoire et immunomodulatrice.

L'invention décrit aussi des procédés pour les préparer, les composés intermédiaires utilisés dans leur préparation ainsi que les compositions pharmaceutiques les contenant.

EP 0 652 214 A1

La présente invention concerne de nouveaux N-aryl-2-cyano-3-hydroxypropène-amides, des procédés pour leur préparation, des compositions pharmaceutiques les contenant et leur utilisation comme médicaments.

L'invention a pour objet de nouveaux dérivés N-aryl 2-cyano 3-hydroxypropène-amides répondant à la formule générale (I):

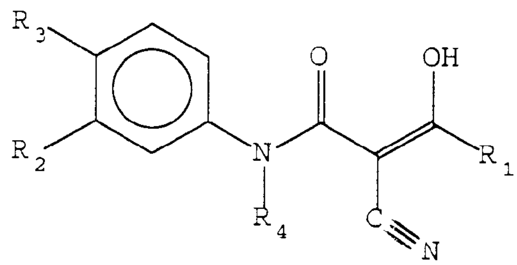

(I)

[dans laquelle

$R_1$ représente un groupe alkyle contenant de 1 à 6 atomes de carbone, un groupe cycloalkyle contenant de 3 à 6 atomes de carbone, un groupe alcényle contenant de 2 à 6 atomes de carbone ou un groupe alcynyle contenant de 2 à 6 atomes de carbone;

$R_2$ et $R_3$, avec les atomes qui les portent, forment un système à noyau carbocyclique ou hétérocyclique, éventuellement substitué; et

$R_4$ représente un atome d'hydrogène ou un groupe alkyle contenant de 1 à 3 atomes de carbone];

et les sels d'addition avec les bases de ces composés.

Il convient de comprendre que l'invention s'étend à toutes les formes tautomères des composés de formule (I).

L'expression "groupe alkyle contenant de 1 à 3 atomes de carbone", telle qu'on l'entend ici, désigne un groupe méthyle, éthyle, propyle ou isopropyle.

L'expression "groupe alkyle contenant de 1 à 6 atomes de carbone", telle qu'on l'entend ici, désigne, par exemple, un groupe méthyle, éthyle, propyle ou isopropyle, ou un groupe butyle, pentyle ou hexyle, linéaire ou ramifié.

L'expression "groupe cycloalkyle contenant de 3 à 6 atomes de carbone", telle qu'on l'entend ici, désigne un groupe cyclopropyle, cyclobutyle, cyclopentyle ou cyclohexyle.

L'expression "atome d'halogène", telle qu'on l'entend ici, englobe un atome de fluor, de chlore, de brome ou d'iode et, de préférence, désigne un atome de fluor, de chlore ou de brome.

L'expression "groupe alcényle" contenant de 2 à 6 atomes de carbone", telle qu'on l'entend ici, désigne de préférence un groupe de formule $-C(CH_3)=CH_2$,

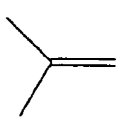 , 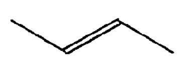 , 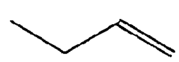 ou 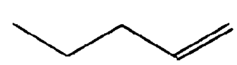 .

L'expression "groupe alcynyle" contenant de 2 à 6 atomes de carbone" désigne, de préférence un groupe de formule

 , 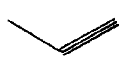 ou 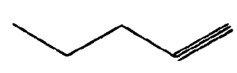 .

L'expression "système à noyau carbocyclique ou hétérocyclique", désigne, de préférence, un système à noyau monocyclique ou bicyclique, en particulier l'un des groupes suivants:

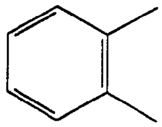 , 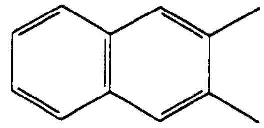 , 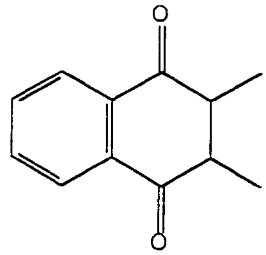 ,

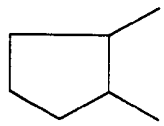 , 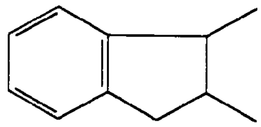 , 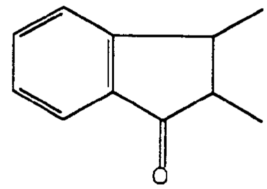 ,

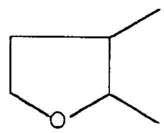 , 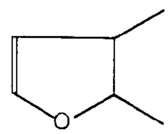 , 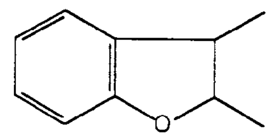 .

La substitution éventuelle du système à noyau carbocyclique ou hétérocyclique est de préférence faite par un ou deux substituants choisis parmi un atome d'halogène, de préférence un atome de fluor ou de chlore, un groupe nitro, un groupe cyano, un groupe alkyle contenant de 1 à 3 atomes de carbone, un groupe alcoxy contenant de 1 à 3 atomes de carbone, un groupe alkylthio contenant de 1 à 3 atomes de carbone, un groupe halogénoalkyle contenant de 1 à 3 atomes de carbone, un groupe alkylcarbonyle contenant de 1 à 3 atomes de carbone dans le groupement alkyle et un groupe alcoxycarbonyle contenant de 1 à 3 atomes de carbone dans le groupement alcoxy. Les systèmes à noyau sont, mieux encore non-substitués ou mono-substitués par un atome d'halogène.

L'expression "groupe alcoxy contenant de 1 à 3 atomes de carbone", telle qu'on l'entend ici, désigne un groupe méthoxy, éthoxy, propoxy, ou isopropoxy.

L'expression "groupe alkylthio contenant de 1 à 3 atomes de carbone", telle qu'on l'entend ici, désigne un groupe méthylthio, éthylthio, propylthio ou isopropylthio.

L'expression "groupe halogénoalkyle contenant de 1 à 3 atomes de carbone", telle qu'on l'entend ici, désigne un groupe alkyle contenant de 1 à 3 atomes de carbone et substitué par un ou plusieurs atomes d'halogène, ces atomes d'halogène pouvant être identiques ou différents. Sont englobés les groupes alkyle monosubstitués par un atome d'halogène, les groupes alkyle complètement substitués par des atomes d'halogène et les groupes alkyle ayant des taux intermédiaires de substitution par des atomes d'halogène. Comme exemples de tels groupes on peut citer les groupes $-CH_2Cl$, $-CF_3$, $-CH_2CF_3$ et $-CF_2CF_3$.

Les sels d'addition de bases peuvent être des sels avec des bases minérales ou organiques, par exemple des sels formés avec des bases minérales telles que les sels de sodium, de potassium, de lithium, de calcium, de magnésium et d'ammonium, ou les sels formés avec des bases organiques telles que la méthylamine, la propylamine, la triméthylamine, la diéthylamine, la triéthylamine, la N,N-diméthyléthanolamine, le tris(hydroxyméthyl)aminométhane, l'éthanolamine, la pyridine, la picoline, la dicyclohexylamine, la morpholine, la benzylamine, la procaïne, la lysine, l'arginine, l'histidine et la N-méthylglucamine.

Les composés préférés selon l'invention sont ceux dans lesquels $R_1$ représente un groupe cycloalkyle contenant de 3 à 6 atomes de carbone ou un groupe alcényle contenant de 2 à 6 atomes de carbone; et $R_2$, $R_3$ et $R_4$ sont tels que définis ci-dessus.

Les composés particulièrement préférés selon l'invention sont ceux dans lesquels $R_1$ représente un groupe cyclopropyle ou un groupe propényle; et $R_2$, $R_3$ et $R_4$ sont tels que définis ci-dessus.

Les composés plus particulièrement préférés selon la présente invention sont ceux dans lesquels $R_1$ représente un groupe cyclopropyle ou un groupe $-C(CH_3)=CH_2$; $R_4$ représente un atome d'hydrogène et $R_2$ et $R_3$ sont tels que définis ci-dessus.

Les composés selon l'invention que l'on préfère tout spécialement sont:

le N-(5-benzofuryl)-2-cyano-3-cyclopropyl-3hydroxy-prop-2-énamide;

le N-[2-(8-chlorodibenzofuryl)]-2-cyano3-cyclopropyl-3-hydroxy-prop-2-énamide;

le N-[2-(8-fluorodibenzofuryl)]-2-cyano-3-cyclopropyl-3-hydroxy-prop-2-énamide;

et leurs sels d'addition avec les bases.

L'invention a également pour objet un procédé de préparation des nouveaux dérivés des N-aryl 2-cyano

3-hydroxy propènamides tels que définis par la formule (I) ci-dessus ainsi que de leurs sels d'addition avec les bases, caractérisé en ce que l'on fait réagir :

a) un composé de formule (II):

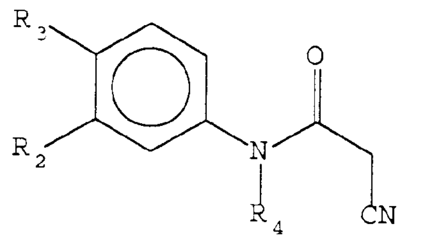

(II)

(dans laquelle $R_2$, $R_3$ et $R_4$ sont tels que définis ci-dessus) avec une base forte, de préférence l'hydrure de sodium (le cas échéant, en présence d'un catalyseur), puis fait réagir le produit avec un composé de formule (III):

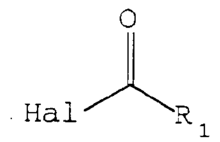

(III)

(dans laquelle Hal représente un atome d'hydrogène et $R_1$ est tel que défini ci-dessus); ou

(b) un composé de formule (II), telle que définie ci-dessus, avec une base forte, de préférence l'hydrure de sodium (le cas échéant, en présence d'un catalyseur), puis fait réagir le produit avec un composé de formule ($III_A$):

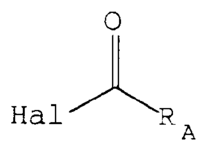

($III_A$)

(dans laquelle Hal représente un atome d'halogène et $R_A$ représente le groupe $R_1$ tel que défini ci-dessus, portant, en outre, un groupe protecteur), pour donner un composé de formule ($I_A$):

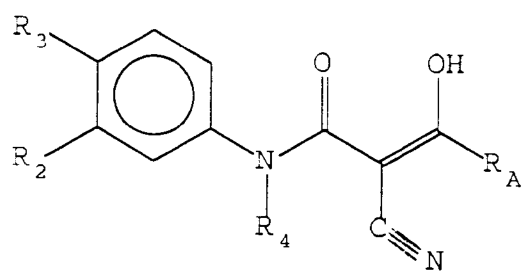

($I_A$)

(dans laquelle $R_A$, $R_2$, $R_3$ et $R_4$ sont tels que définis ci-dessus), puis élimine le groupe protecteur, pour obtenir le composé de formule ($I_A$) correspondant, dans lequel $R_A$ représente un groupe $R_1$ tel que défini ci-dessus.

Pour chacun des procédés ci-dessus, on peut, le cas échéant, transformer ensuite le composé de formule (I) ainsi obtenu en un de ses sels d'addition avec les bases par des procédés classiques.

La réaction entre le composé de formule (II) et la base forte, par exemple l'hydrure de sodium, est de préférence effectuée en présence d'un solvant organique anhydre tel que le tétrahydrofurane ou le dichlorométhane et, le cas échéant, en présence d'un catalyseur capable de solvater la base forte, tel que, par exemple, l'imidazole.

La réaction entre le produit de la réaction du composé de formule (II) et la base forte et le composé de formule (III) ou ($III_A$) est de préférence réalisée en présence d'imidazole, dans un solvant organique anhydre

tel que le tétrahydrofurane ou le dichlorométhane, à température ambiante ou à basse température.

Le composé de formule (III) ou ($III_A$) est de préférence un chlorure d'acide ou un fluorure d'acide. Cependant, on peut utiliser d'autres dérivés activés d'acides carboxyliques. Comme exemple de composé de formule (III), on peut mentionner le fluorure de propynyle qui peut être, par exemple, préparé par action de l'acide propiolique sur du fluorure de benzoyle puis distillé pour donner le mélange réactionnel résultant.

Lorsque le groupe $R_A$ représente un groupe $R_1$ portant, en outre, un groupe protecteur, ce groupe protecteur peut être, par exemple, un groupe arylséléno tel que phénylséléno. L'élimination de ce groupe protecteur peut être réalisée, par exemple, par oxydation, au moyen, par exemple, d'un peroxyde tel que le peroxyde d'hydrogène, en l'absence de solvant ou en présence d'un mélange de solvants organiques tel que, par exemple, un système méthanol/dichlorométhane.

L'invention a également pour objet les composés de formule (II), telle que définie ci-dessus.

Les composés de formule (II) peuvent être préparés par réaction d'un composé de formule (IV) :

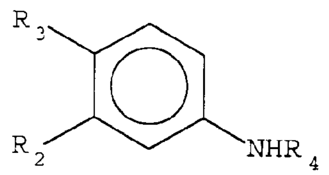

(IV)

(dans laquelle $R_2$, $R_3$ et $R_4$ sont tels que définis ci-dessus) avec de l'acide cyanoacétique, de préférence en présence de dicyclohexylcarbodiimide ou de pentachlorure de phosphore, et en présence d'un solvant organique anhydre tel que le tétrahydrofurane ou le dichlorométhane.

Les composés de formule (IV) qui ne sont pas déjà connus peuvent être préparés par réduction des composés nitro correspondants.

Les composés de formule (I) ont un caractère acide.

Les sels d'addition avec les bases des composés de formule (I) peuvent être avantageusement préparés par réaction, dans des proportions approximativement stoechiométriques d'une base minérale ou organique avec le composé de formule (I). Les sels peuvent être préparés sans isolement intermédiaire du composé acide correspondant.

Les composés selon l'invention possèdent des propriétés pharmacologiques très intéressantes. Leur remarquable activité anti-inflammatoire est tout particulièrement intéressante. Ils inhibent à la fois la réponse inflammatoire provoquée par des agents irritants et des réactions d'hypersensibilité retardées, en empêchant l'activation des cellules immunitaires par un antigène spécifique.

Ces propriétés sont illustrées plus en détail dans la partie expérimentale.

Les composés de formule (I), et leurs sels d'addition avec les bases pharmacologiquement acceptables, justifient leur utilisation comme médicaments.

L'invention a ainsi également pour objet l'application à titre de médicaments, des composés de formule (I), tels que définis ci-dessus, et de leurs sels d'addition avec les bases pharmacologiquement acceptables.

Ces médicaments trouvent leur emploi, par exemple, dans le traitement de la polyarthrite rhumatoïde, des maladies inflammatoires chroniques d'origine immunitaire ou non (par exemple la réaction du greffon contre l'hôte, les réactions de transplantation, l'uvéite), le cancer, le diabète, la prévention du rejet aigu ou chronique suivant les transplantations d'organes et la resténose après angioplastie.

La dose habituelle varie en fonction du composé utilisé, du patient traité et de la maladie en question et peut être, par exemple, de 0,1 mg à 200 mg par jour, par voie orale.

L'invention a encore pour objet les compositions pharmaceutiques comprenant, comme substance active, au moins un composé de formule (I), telle que définie ci-dessus, ou un de ses sels d'addition avec les bases pharmacologiquement acceptables, en association avec un ou plusieurs diluants, véhicules et/ou excipients pharmacologiquement acceptables.

A titre de médicaments, les composés de formule (I) et leurs sels d'addition avec les bases pharmaceutiquement acceptables peuvent être incorporés dans des compositions pharmaceutiques destinées à être administrées par voie orale, rectale ou parentérale.

Ces compositions pharmaceutiques peuvent être, par exemple, solides ou liquides, et se présenter sous les formes couramment utilisées en médecine humaine, comme par exemple, les comprimés simples ou dragéifiés, les gélules, les capsules, les granulés, les suppositoires, les préparations injectables ; elles peuvent être préparées selon des procédés classiques. Le ou les principes actifs peuvent être incorporés à des excipients que l'on utilise couramment dans les compositions pharmaceutiques, tels que le talc, la gomme arabique, le lactose, l'amidon, le stéarate de magnésium, le beurre de cacao, les véhicules aqueux ou non aqueux,

les substances grasses d'origine animale ou végétale, les dérivés paraffiniques, des glycols, les divers agents mouillants, dispersants ou émulsifiants, et les conservateurs.

L'invention a enfin pour objet une méthode de traitement de la polyarthrite rhumatoïde, des maladies inflammatoires chroniques d'origine immunitaire ou non immunitaire et du cancer chez l'homme ou l'animal, qui comprend l'administration au sujet d'une quantité efficace d'un composé de formule (I), telle que définie ci-dessus, ou d'un de ses sels d'addition avec les bases pharmacologiquement acceptables.

L'invention est illustrée plus en détail par les exemples non limitatifs suivants.

**EXEMPLE 1 : N-[5-(2,3-dihydrobenzofuryl)]-2-cyano-3-cyclopropyl-3hydroxy-prop-2-énamide.**

Stade 1: 5-nitro-2,3-dihydrobenzofurane

On ajoute goutte à goutte une solution de chlorure d'acétyle (14,37g, 13,02 ml, 103 mmoles, 1,1 équ.) dans de l'acétonitrile (20 ml) à un mélange de nitrate d'argent (31,11 g, 103 mmoles, 1,1 équ.) et de 2,3-dihydrobenzofurane (20,0 g, 167 mmoles) dans de l'acétonitrile (120 ml) à 5-10°C. Après une heure d'agitation à 5-10°C, on ajoute de l'eau (180 ml). Lorsqu'on atteint la température ambiante, on ajoute encore 50 ml d'eau. Après avoir agité le mélange pendant 3 h ½, on le filtre et on lave le résidu avec de l'éther (5 x 50 ml). La partie aqueuse est extraite avec de l'éther (3 x 250 ml). Les extraits organiques réunis sont lavés avec de l'eau (3 x 250 ml) et de la saumure (250 ml), séchés ($MgSO_4$), filtrés et évaporés, pour donner 22,85 g d'un solide marron contenant deux composés nitro isomères.

Une chromatographie sur colonne éclair (éluant: 5-60% d'acétate d'éthyle/40-60% d'éther de pétrole) donne 7,83 g (29%) de produit.

RMN $^1H$ ($CDCl_3$) 3,31 (2H, t); 4,75 (2H, t); 6,82 (1H, d); 8,07-8,13 (2H,m).

Stade 2 : 5-amino-2,3-dihydrobenzofurane

On agite sous hydrogène, dans de l'éthanol (100 ml), pendant 7 heures, un mélange de 5-nitro-2,3dihydrobenzofurane (7,83 g, 47,4 mmoles) et d'oxyde de platine (60 mg, 5% en moles). On filtre la suspension à travers de la Célite et on élimine le solvant pour obtenir 6,34 g de cristaux marron foncé. Une chromatographie sur colonne éclair (5-100% d'acétate d'éthyle/40-60% d'éther de pétrole) donne 5,71 g (89%) de produit sous forme de cristaux marron.

RMN $^1H$ ($CDCl_3$) 3,12 (2H, t); 3,32 (2H, s large); 4,49 (2H, t); 6,45 (1H, dd); 6,58-6,62 (2H, m).

Stade 3 : N-[5-(2,3-dihydrobenzofuryl)]-cyanoacétamide

On ajoute de l'acide cyanoacétique (5,39 g, 63,4 mmoles, 1,5 équ.) par portions, en l'espace de 30 minutes, à une suspension de pentachlorure de phosphore (13,20 g, 63,4 mmoles, 1,5 équ.) dans $CH_2Cl_2$ (75 ml). La solution est chauffée au reflux pendant 1 heure avant que l'on ajoute une solution d'amine (5-amino-2,3-dihydrobenzofurane) (5,71 g, 42,3 mmoles) dans $CH_2Cl_2$ (15 ml). On porte le mélange au reflux pendant une 1 h ½, puis on le laisse refroidir à la température ambiante. On ajoute avec précaution une solution aqueuse saturée de bicarbonate de sodium (125 ml) et on agite le mélange pendant une heure, puis on le filtre. Le résidu est lavé avec de l'eau (2 x 50 ml) et séché sous pression réduite à 50°C, pour donner 6,51 g (76%) de solide beige.

RMN $^1H$ ($CDCl_3/CD_3OD$) 3,20 (2H, t); 3,53 (2H, s); 4,57 (2H, t); 6,72 (1H, d); 7,09 (1H, dd); 7,47 (1H, d); 9,25 (1H, s)

Stade 4 : N-[5-(2,3-dihydrobenzofuryl)]2-cyano-3-cyclopropyl-3-hydroxy-prop-2-énamide

On ajoute un amide (N-[5-(2,3-dihydrobenzofuryl)]-cyanoacétamide) (6,17 g, 30,5 mmoles), par portions, en l'espace de 30 minutes, à une suspension d'hydrure de sodium (dispersion à 80% dans de l'huile minérale, 2,75 g, 91,5 mmoles, 3 équ.) dans le tétrahydrofurane (150 ml). On agite la suspension pendant une heure avant d'ajouter goutte à goutte une solution de chlorure de cyclopropylcarbonyle (4,15 ml, 45,8 mmoles, 1,5 équ.) dans du THF (30 ml). Au bout d'une heure, on ajoute 1,38 ml supplémentaires (0,5 équ.) de chlorure de cyclopropylcarbonyle et, au bout de 2 heures, 458 mg de plus (0,5 équ.) d'hydrure de sodium. Au bout de 2 h ½, on stoppe la réaction en ajoutant avec précaution de l'acide acétique (5,24 ml, 91,5 mmoles, 3 équ.). Le mélange est agité pendant 1 h ½, puis ajouté goutte à goutte à un système d'acide chlorhydrique concentré (30 ml) et d'eau glacée (470 ml) sous vive agitation. Le mélange est agité puis filtré. Le résidu est lavé avec de l'éther (20 ml) pour donner 1,39 g de poudre crème. Le filtrat est extrait avec 400 ml d'acétate d'éthyle. La

phase organique est lavée à l'eau (3 x 300 ml) et à la saumure (100 ml), puis séchée ($MgSO_4$), filtrée et évaporée, pour donner 3,60 g (44%) de poudre crème.

RMN $^1H$ ($CDCl_3$) 1,08-1,57 (4H, m); 2,08-2,20 (1H, m); 3,23 (2H, t); 4,60 (2H, t); 6,76 (1H, d); 7,07 (1H, dd); 7,34 (1H, d); 7,43 (1H, s); 1,60 (1H, s).

**EXEMPLE 2 : N-(5-benzofuryl)-2-cyano-3-cyclopropyl-3-hydroxyprop-2-énamide**

Stade 1 : 5-nitrobenzofurane

On ajoute du N-bromosuccinimide (5,28 g, 29,7 mmoles, 0,98 équ.) et du peroxyde de benzoyle (quelques cristaux) à une solution de 5-nitro-2,3-dihydrobenzofurane (5,0 g, 30,3 mmoles) dans du tétrachlorure de carbone (250 ml). Le mélange est porté au reflux pendant 3 h ¼, refroidi à la température ambiante, puis filtré et évaporé, pour donner 5,47 g de poudre crème. Une chromatographie sur colonne éclair (2-30% d'acétate d'éthyle dans de l'éther de pétrole) donne 4,9 g de produit.

RMN $^1H$ (CDCl3) 6,94 (1H, d); 7,59 (1H, d); 7,79 (1H, d); 8,24 (1H, dd); 8,56 (1H, d).

Stade 2 : 5-aminobenzofurane

On ajoute goutte à goutte, en l'espace d'une heure, une solution d'acide chlorhydrique concentrée (2,8 ml) dans de l'éthanol aqueux à 50% (50 ml) à une suspension au reflux de 5-nitrobenzofurane (4,55 g, 27,9 mmoles) et de poudre de fer (4,67 g, 83,7 mmoles, 3 équ.) dans de l'éthanol aqueux à 50% (200 ml). On porte le mélange au reflux pendant 3 heures, puis on le laisse refroidir à la température ambiante. On ajoute une solution aqueuse d'hydroxyde de sodium pour alcaliniser le mélange, que l'on filtre. Le résidu est agité avec de l'acétate d'éthyle au reflux (2 x 100 ml) et filtré. Le filtrat est évaporé et la phase aqueuse reprise avec de l'acétate d'éthyle (400 ml) et de l'eau (250 ml). Les phases sont séparées et la phase organique est lavée avec de l'eau (3 x 250 ml) et de l'eau salée (250 ml), puis séchée ($MgSO_4$), filtrée et évaporée pour donner 3,71 g de liquide marron. Une chromatographie sur colonne éclair (5-50% d'acétate d'éthyle dans de l'éther de pétrole) donne 3,19 g (86%) de liquide marron-rouge foncé.

RMN $^1H$ ($CDCl_3$) 3,51 (2H, s large); 6,56 (1H, d); 6,61 (1H, dd); 6,77 (1H, d); 7,26 (1H, d); 7,49 (1H, d).

Stade 3: N-(5-benzofuryl)cyanoacétamide

Le procédé est identique à celui de l'exemple 1, stade 3, avec un rendement de 59%.

RMN $^1H$ ($d^6$-DMSO) 3,96 (2H, s); 7,01 (1H, d); 7,40 (1H, dd); 7,60 (1H, d); 8,00-8,03 (2H, m); 10,40 (1H, s).

Stade 4 : N-(5-benzofuryl)-2-cyano-3cyclopropyl-3-hydroxyprop-2-énamide

Le procédé est identique à celui de l'exemple 1, stade 4, avec un rendement de 41%.

RMN $^1H$ ($d^6$-DMSO) 1,15-1,23 (4H, m); 2,16-2,25 (1H, m); 7,01 (1H, d); 7,42 (1H,dd) ; 7,61 (1H, d); 7,84 (1H, d); 8,05 (1H, d); 10,40 (1H, s large).

**EXEMPLE 3 : N-[5-(2,3-dihydrobenzofuryl)-2-cyano-3-hydroxy-3-isopropényl-prop-2-énamide**

Le procédé est identique à celui de l'exemple 1, sauf que l'on utilise du chlorure de méthacryloyle à la place du chlorure de cyclopropylcarbonyle dans le stade 4.

**EXEMPLE 4 : N-(2-anthracényl)-2-cyano-3-cyclopropyl-3-hydroxy-prop-2-énamide**

Stade 1 : N-(2-anthracényl)cyanoacétamide

Le procédé est identique à celui de l'exemple 1, stade 3.

Stade 2 : N-(2-anthracényl)-2-cyano-3cyclopropyl-3-hydroxyprop-2-énamide

Le procédé est identique à celui de l'exemple 1, stade 4, avec un rendement de 37%.

RMN $^1H$ ($d^6$-DMSO) 1,00-1,09 (4H, m); 2,19-2,29 (1H, m); 7,47-7,67 (3H, m); 8,02-8,13 (3H, m); 8,39 (1H, d); 8,51 (2H, d) ; 11,29 (1H, s)

**EXEMPLE 5 : N-[2-anthraquinoyl]-2-cyano-3-cyclopropyl-3-hydroxyprop-2-énamide**

Stade 1 : N-(2-anthraquinoyl)cyanoacétamide

Le procédé est identique à celui de l'exemple 1, stade 3.

Stade 2 : N-[2-anthraquinoyl]-2-cyano-3cyclopropyl-3-hydroxyprop-2-énamide

Le procédé est identique à celui de l'exemple 1, stade 4.

RMN $^1H$ ($d^6$-DMSO) 0,91-1,11 (4H, m); 2,17-2,29 (1H, m); 7,88-7,98 (3H, m); 8,19-8,25 (3H, m); 8,45 (1H, d); 12,13 (1H, s).

**EXEMPLE 6 : N-[2-(8-chlorodibenzofuryl)-2-cyano-3-cyclopropyl-3hydroxy-prop-2-énamide**

Stade 1 : 2-bromo-4-chloro-1-(4'-nitrophénoxy)-benzène

On ajoute goutte à goutte, en l'espace de 10 minutes, une solution de 4-fluoronitrobenzène (14,10 g, 100 mmoles) dans du diméthylsulfoxyde (30 ml) à une suspension de 2-bromo-4-chlorophénol (20,75 g, 100 mmoles) et de carbonate de potassium (15,20 g, 110 moles, 1,1 équ.) dans 130 ml de DMSO. On chauffe le mélange à 70°C pendant 20 heures, puis on le laisse refroidir à la température ambiante. On ajoute de l'eau (300 ml) et de l'acétate d'éthyle (300 ml) et les phases se séparent. La phase organique est lavée avec de l'eau (4 x 200 ml), de la saumure (75 ml), séchée ($MgSO_4$), filtrée et évaporée, pour donner 31,7 g (97%) de solide orange.

RMN $^1H$ ($CDCl_3$) 6,96 (2H, d); 7,01 (1H, d); 7,37 (1H, dd); 7,70 (1H, d); 8,22 (2H, d).

Stade 2 : 2-chloro-8-nitrodibenzofurane

On porte au reflux un mélange de 2-bromo-4-chloro-1-(4'-nitrophénoxy)-benzène (31,4 g, 95,7 mmoles), de carbonate de sodium (12,2 g, 115 mmoles, 1,2 équ.) et d'acétate de palladium (107 mg, 478 moles, 0,5%) dans du N,N-diméthylacétamide (250 ml) pendant 3 heures, puis on le laisse refroidir à la température ambiante. On ajoute de l'acétate d'éthyle ( 1 l) et de l'eau (700 ml) et les phases se séparent. Les phases organiques sont lavées avec de l'eau (3 x 250 ml), et de la saumure (100 ml), séchées ($MgSO_4$), filtrées et évaporées. La phase aqueuse est encore extraite avec $CH_2Cl_2$ (700 ml). Les phases organiques sont séchées, évaporées et réunies avec le résidu ci-dessus. Une chromatographie sur colonne éclair donne 21,50 g (91%) de solide jaune.

RMN $^1H$ ($CDCl_3$) 7,55 (2H, m); 7,67 (1H, d); 8,01 (2H, d); 8,44 (1H, dd); 8,55 (1H, d).

Stade 3 : 2-amino-8-chlorodibenzofurane

Le procédé est identique à celui de l'exemple 1, stade 2, avec un rendement de 74%.

RMN $^1H$ ($CDCl_3$) 3,73 (2H, s large); 6,85 (1H, dd); 7,18 (1H, d); 7,32-7,46 (3H, m); 7,82 (1H, d).

Stade 4 : N-[2-(8-chlorodibenzofuryl)]-cyanoacétamide

Le procédé est identique à celui de l'exemple 1, stade 3.

RMN $^1H$ ($d^6$-DMSO) 4,02 (2H, s); 7,57-7,80 (4H, m); 8,35 (1H, d); 8,44 (1H, d); 10,61 (1H, s).

Stade 5 : N-[2-(8-chlorodibenzofuryl)]-2cyano-3-cyclopropyl-3-hydroxy-prop-2-énamide

Le procédé est identique à celui de l'exemple 1, stade 4. Une purification par chromatographie sur colonne ($CH_2Cl_2$ > acétate d'éthyle > $(CH_3)_2CO$ > $(CH_3)_2CO$/MeOH 80:20) donne le composé attendu avec un rendement de 33%.

RMN $^1H$ ($d^6$-DMSO) 1,00-1,06 (4H, m); 2,17-2,27 (1H, m); 7,57 (1H, dd); 7,71 (2H, s); 7,78 (1H, d); 8,32 (1H, dd); 11,19 (1H, s).

**EXEMPLE 7** : **N-[2-(8-fluorodibenzofuryl)]-2-cyano-3-cyclopropyl-3hydroxy-prop-2-énamide**

Stade 1 : 2-bromo-4-fluoro-1-(4'-nitrophénoxy)benzène

Le procédé est identique à celui de l'exemple 6, stade 1, avec un rendement de 91%.
RMN $^1H$ ($CDCl_3$) 6,94 (2H, d); 7,12-7,18 (2H, m); 7,44 (1H, m); 8,21 (2H, d).

Stade 2 : 2-fluoro-8-nitrodibenzofurane

Le procédé est identique à celui de l'exemple 6, stade 2, avec un rendement de 68%.
RMN $^1H$ ($CDCl_3$) 7,30 (1H, dt); 7,60 (1H, dd); 7,67 (1H, d); 7,70 (1H, dd); 8,43 (1H, dd); 8,85 (1H, d).

Stade 3 : 2-amino-8-fluorodibenzofurane

Le procédé est identique à celui de l'exemple 1, stade 2, avec un rendement de 99%.
RMN $^1H$ ($CDCl_3$) 3,72 (2H, s); 6,85 (1H, dd); 7,12-7,18 (2H, m); 7,36 (1H, d); 7,43 (1H, dd); 7,50 (1H, dd).

Stade 4 : N-[2-(8-fluorodibenzofuryl)]cyanoacétamide

Le procédé est identique à celui de l'exemple 1, stade 3, avec un rendement de 68%.
RMN $^1H$ ($d^6$-DMSO) 4,05 (2H, s); 7,41 (1H, dt); 7,63-7,79 (3H, m); 8,07 (1H, dd); 8,46 (1H, d); 10,84 (1H, s).

Stade 5 : N-[2-(8-fluorodibenzofuryl)]-2-cyano-3-cyclopropyl-3-hydroxy-prop-2-énamide

Le procédé est identique à celui de l'exemple 6, stade 5, avec un rendement de 62%.
RMN $^1H$ ($d^6$-DMSO) 1,16 (4H, m); 2,17-2,30 (1H, m); 7,42 (2H, dt); 7,65-7,81 (3H, m); 8,06 (1H, dd); 8,32 (1H, d); 10,77 (1H, s).

**EXEMPLE 8** : **N-[2-(7-iodofluorényl)]-2-cyano-3-cyclopropyl-3-hydroxy-prop-2-énamide**

Stade 1 : 2-iodo-7-nitrofluorène

On agite un mélange de 2-nitrofluorène (2,0 g, 9,47 mmoles) et d'iode (1,20 g, 4,73 mmoles, 0,5 équ.) dans de l'acide acétique (100 ml) pendant 15 minutes. On ajoute de l'acide sulfurique concentré (6 ml) et du nitrite de sodium (0,65 g, 9,47 mmoles) et on porte le mélange au reflux pendant 1 heure. On verse le mélange chaud sur de la glace (50 ml) et on ajoute de l'eau (300 ml). On filtre la suspension et on lave le solide jaune (2 x 50 ml d'eau) et on le sèche sous pression réduite, pour obtenir 3,02 g de produit.
RMN $^1H$ ($d^6$-DMSO) 4,12 (2H, s); 7,87 (1H, dd); 7,95 (1H, d); 8,13 (1H, d); 8,21 (1H, d); 8,34 (1H, dd); 8,50 (1H, d).

Stade 2 : 2-amino-7-iodofluorène

Le procédé est identique à celui de l'exemple 2, stade 2, avec un rendement de 78%.
RMN $^1H$ ($CDCl_3$) 3,77 (4H, m); 6,71 (1H, dd); 6,85 (1H, d); 7,37 (1H, d); 7,53 (1H, d); 7,62 (1H, dd). 7,78 (1H, d).

Stade 3 : N-[2-(7-iodofluorényl)]cyanoacétamide

Le procédé est identique à celui de l'exemple 1, stade 3, avec un rendement de 70%.
RMN $^1H$ ($d^6$-DMSO) 3,97 (4H, s); 7,56 (1H, dd); 7,70 (1H, d); 7,77 (1H, d); 7,88-7,98 (3H, m); 10,46 (1H, s).

Stade 4 : N-[2-(7-iodofluorényl)]-2-cyano-3-cyclopropyl-3-hydroxy-prop-2-énamide

Le procédé est identique à celui de l'exemple 1, stade 4, avec un rendement de 58%.
RMN $^1H$ ($d^6$-DMSO) 1,05-1,10 (4H, m); 2,15-2,29 (1H, m); 3,95 (2H, s); 7,54 (1H, dd); 7,62-7,74 (2H, m); 7,77-7,90 (2H, m); 7,97 (1H, d); 10,87 (1H, s).

Les résultats spectraux, les rendements, les points de fusion et les résultats analytiques des exemples 1 à 8 sont indiqués sur le tableau I.

TABLEAU I

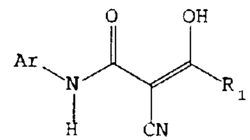
(I)

| Ex | Ar- | $-R_1$ | Rdt % | Pt de F°C | IR $cm^{-1}$ KBr | RMN $^1H$ δ | Formule M.M | Analyse % Calc. | Trouvé |
|---|---|---|---|---|---|---|---|---|---|
| 1 | 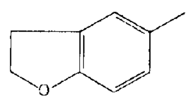 | 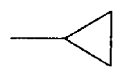 | 44 | 143-144 | 3270, 2200, 1610, 1570, 1540, 1480, 1430, 1410, 1340, 1300, 1220, 1200, 1060, 975, 890, 870, 795. | $CDCl_3$ 1.08-1.57 (4H,m);2.08-2.20(1H,m); 3.23(2H,t); 4.60(2H,t); 6.76(1H,d); 7.07(1H,dd); 7.34(1H,d); 7.43(1H,s); 16.0(1H,s). | $C_{15}H_{14}N_2O_3$ 270.28 | C 66.65 H 5.22 N 10.37 | 66.59 5.33 10.45 |
| 2 | 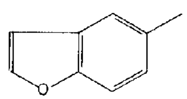 | 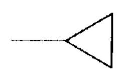 | 41 | 137-140 | 3260, 2910, 2210, 1625, 1580, 1540, 1470, 1410, 1360, 1270, 775. | DMSO-$d_6$ 1.15-1.23 (4H,m);2.16-2.25(1H,m); 7.01(1H,d); 7.42(1H,dd); 7.61(1H,d); 7.84(1H,d); 8.05(1H,d); 10.40(1H,s). | $C_{15}H_{12}N_2O_3$ 268.26 | C 67.15 H 4.51 N 10.44 | 67.30 4.73 10.12 |
| 3 | 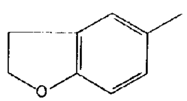 | 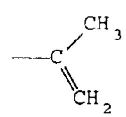 | 36 | 133-134 | 3280, 2200, 1680, 1540, 1485, 1370, 1320, 1275, 1225, 1200, 1100, 1010, 975, 910, 870, 790, 780, 635. | DMSO-$d_6$ 2.00(3H,s); 3.21(2H,m); 4.66(2H,m); 5.76(2H,d); 6.78(1H,m); 7.21(1H,m); 7.42(1H,m); 10.38(1H,s). | $C_{15}H_{14}N_2O_3$ 270.28 | C 66.65 H 5.22 N 10.37 | 66.00 5.20 10.21 |

TABLEAU I suite

| Ex | Ar- | $-R_1$ | Rendt % | Pt de F°C | IR $cm^{-1}$ KBr | RMN $^1H$ δ | Formule M.M | Analyse % Calc. | Trouvé |
|---|---|---|---|---|---|---|---|---|---|
| 4 | 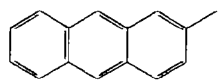 | 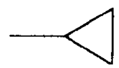 | 37 | 208-209 | 3300, 2210, 1580, 1540, 1525, 1455, 1415, 1360, 1345, 1280, 1255, 890, 740. | DMSO-$d_6$ 1.00-1.09 (4H,m);2.19-2.29(1H,m); 7.47-7.67 (3H,m);8.02-8.13(3H,m); 8.39(1H,d); 8.51(2H,d); 11.29(1H,s). | $C_{21}H_{16}N_2O_2$ 328.37 | C 76.81<br>H 4.91<br>N 8.53 | 76.7<br>0<br>4.98<br>8.42 |
| 5 | 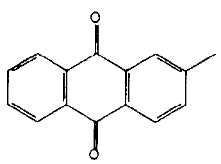 | 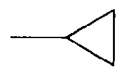 | | >220 | 3300, 2220, 1670, 1635, 1585, 1540, 1420, 1330, 1290, 965, 930, 890, 705. | DMSO-$d_6$ 0.91-1.11 (4H,m);2.17-2.29(1H,m); 7.88-7.98 (3H,m);8.19-8.25(3H,m); 8.45(1H,d); 12.13(1H,s). | $C_{21}H_{14}N_2O_4$ 358.34 | C 70.38<br>H 3.94<br>N 7.82 | 69.6<br>8<br>3.91<br>7.90 |
| 6 | 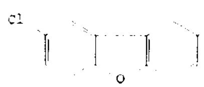 | 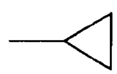 | 33 | >200 decomp. | 3310, 2205, 1630, 1575, 1530, 1440, 1405, 1350, 1260, 1245, 1195, 1060, 895, 865, 820, 805. | DMSO-$d_6$ 1.00-1.06 (4H,m);2.17-2.27(1H,m); 7.57(1H,dd); 7.71(2H,s); 7.78(1H,d); 8.32(1H,dd); 11.19(1H,s). | $C_{19}H_{13}ClN_2O_3$ 352.78 | C 64.69<br>H 3.71<br>N 7.94<br>Cl10.05 | 64.6<br>9<br>3.93<br>7.83<br>9.90 |

TABLEAU I suite

| Ex | Ar- | $-R_1$ | Rendt % | Pt de FC° | IR $cm^{-1}$ KBr | RMN $^1H$ δ | Formule M.M | Analyse % Calc. | Trouvé |
|---|---|---|---|---|---|---|---|---|---|
| 7 | 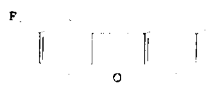 | 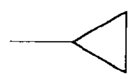 | 62 | >250 | 3300, 2210, 1590, 1555, 1480, 1450, 1410, 1355, 1310, 1165. | $d^6$-DMSO 1.10-1.16 (4H,m);2.17-2.30(1H,m); 7.42(2H,dt); 7.65-7.81 (3H,m);8.06 (1H,dd);8.32 (1H,d);10.77 (1H,s). | $C_{19}H_{13}FN_2O_3$ 336.31 | C 67.85<br>H 3.90<br>N 8.33<br>F 5.65 | 67.56<br>4.03<br>8.21<br>5.62 |
| 8 | 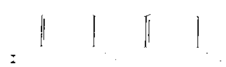 | 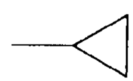 | 58 | >250 decomp. | 3280, 2205, 1540, 1530, 1480, 1430, 1400, 1345, 1265, 1250, 885, 800. | $d^6$-DMSO 1.05-1.10 (4H,m);2.15-2.29(1H,m); 3.95(2H,s); 7.54(1H,dd); 7.62-7.74 (2H,m);7.77-7.90(2H,m); 7.97(1H,d); 10.87(1H,s). | $C_{20}H_{15}IN_2O_2$ 442.26 | C 54.32<br>H 3.42<br>N 6.33<br>I 28.69 | 54.47<br>3.49<br>6.30<br>28.11 |
| | | | | | | | | | |

**EXEMPLE 9** :

On prépare des comprimés correspondants à la formule suivante:

| | |
|---|---|
| Composé de l'exemple 1 | 20 mg |
| Excipient pour 1 comprimé, q.s.p. | 150 mg |

(Détail de l'excipient: lactose, amidon, talc, stéarate de magnésium).

ACTIVITE PHARMACOLOGIQUE

Méthodes d'essai biochimiques

Oedème de la patte du rat provoqué par la carragénine (PO-R): essai 1

Une heure après l'administration orale des composés étudiés ou du véhicule témoin à des groupes de rats (n=6-12, CFHB mâles, gamme de poids 160-180 g), on injecte 1 mg de carragénine dissoute dans 0,2 ml de sérum physiologique dans la patte arrière. Les pattes contralatérales reçoivent des injections témoins de sérum physiologique. Les réactions d'oedème de la patte sont évaluées 3 heures plus tard. Oedème de la patte de la souris par hypersensibilité de type retardé (DTH-M): essai 2

Des groupes de souris (n=8-10, CD-1 mâles, gamme de poids 25-30 g) sont sensibilisés par une injection sous-cutanée de 1 mg de sérum-albumine bovine méthylée (MBSA) dans des volumes de 0,2 ml d'émulsion d'adjuvant complet de Freund (FCA) dans du sérum physiologique. Les groupes témoins négatifs reçoivent des injections d'émulsion de FCA dans du sérum physiologique. Les réactions d'oedèmes de la patte DTH sont évaluées 24 heures après le défi dans la patte arrière droite avec 0,1 mg de MBSA dans des volumes de 0,5 ml de sérum physiologique, le septième jour après la sensibilisation. Les pattes contralatérales reçoivent des injections témoins de sérum physiologique. Les composés étudiés et les véhicules témoins sont administrés oralement une fois par jour aux jours 4, 5, 6, et deux fois par jour le 7ème jour, une heure avant et 6 heures après le défi au MBSA.

Oedème de la patte du rat par hypersensibilité de type retardé (DTH-R): essai 3

Des groupes de rats (n=18-12, CFHB mâles, gamme de poids 160-180 g) sont sensibilisés par injection sous-cutanée à la base de la queue avec des volumes de 0,1 ml de FCA. Les groupes témoins négatifs reçoivent des adjuvants incomplets de Freund. Les réactions d'oedème des pattes DTH sont évaluées 24 heures après le défi dans la patte arrière droite avec 0,1 mg de MBSA dans 0,4 mg d'antigène d'extrait de Mycobacterium tuberculosis dans des volumes de 0,2 ml de solution saline, le septième jour après la sensibilisation. Les pattes contralatérales reçoivent des injections témoins de sérum physiologique. Les composés étudiés sont administrés oralement une fois par jour aux jours 4, 5, 6, et deux fois par jour le 7ème jour, une heure avant et 6 heures après le défi antigénique.

Les résultats de ces essais sont indiqués dans le tableau II, qui donne le pourcentage d'inhibition de la formation de l'oedème. Les doses sont exprimées en unités mg/kg, p.o.

TABLEAU II

| Exemple | Essai 1 | | Essai 2 | | Essai 3 | |
|---|---|---|---|---|---|---|
| | % inhibition | Dose | % inhibition | Dose | % inhibition | Dose |
| 1 | 13 | 50 | -9 | 100 | 51 | 50 |
| 2 | 20 | 50 | -4 | 100 | 69 | 50 |
| 3 | | | 2 | 100 | | |
| 4 | -20 | 50 | 25 | 100 | 33 | 50 |
| 5 | 10 | 50 | 34 | 100 | -11 | 50 |
| 6 | 15 | 50 | 45 | 100 | 59 | 50 |
| 7 | 11 | 50 | 15 | 100 | 54 | 50 |
| 8 | 15 | 50 | 33 | 100 | 56 | 50 |

## Revendications

1. Composés de formule générale (I):

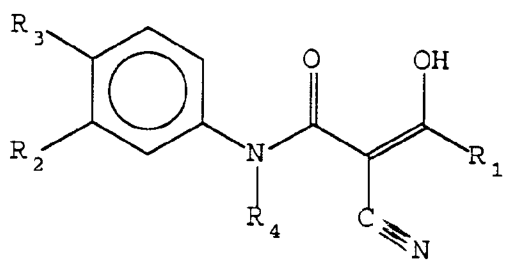

(I)

[dans laquelle
$R_1$ représente un groupe alkyle contenant de 1 à 6 atomes de carbone, un groupe cycloalkyle contenant de 3 à 6 atomes de carbone, un groupe alcényle contenant de 2 à 6 atomes de carbone ou un groupe alcynyle contenant de 2 à 6 atomes de carbone;
$R_2$ et $R_3$, avec les atomes qui les portent, forment un système à noyau carbocyclique ou hétérocyclique, éventuellement substitué; et
$R_4$ représente un atome d'hydrogène ou un groupe alkyle contenant de 1 à 3 atomes de carbone]; leurs tautomères et les sels d'addition avec les bases de ces composés.

2. Composés selon la revendication 1, dans lesquels $R_2$ et $R_3$, avec les atomes qui les portent, forment un système à noyau carbocyclique ou hétérocyclique, éventuellement substitué, choisi dans le groupe comprenant

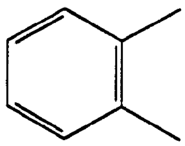 , 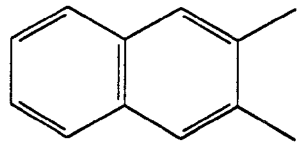 , 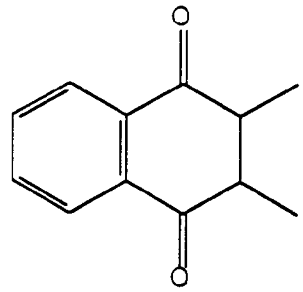 ,

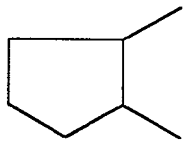 , 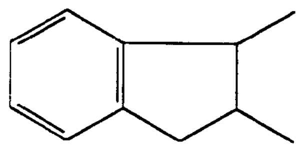 , 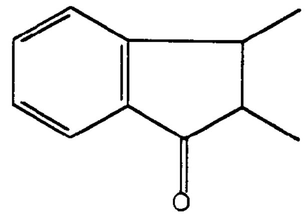 ,

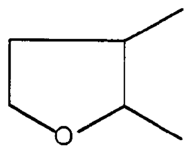 , 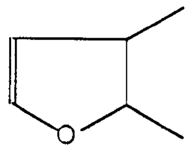 , 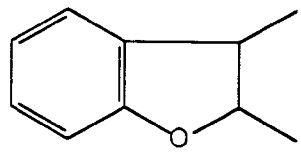 .

3. Composés selon la revendication 1 ou 2, dans lesquels $R_1$ représente un groupe cycloalkyle contenant de 3 à 6 atomes de carbone ou un groupe alcényle contenant de 2 à 6 atomes de carbone; et $R_2$, $R_3$ sont tels que définis dans la revendication 2, et $R_4$ est tel que défini dans la revendication 1.

4. Composés selon la revendication 3, dans lesquels $R_1$ représente un groupe cyclopropyle ou un groupe propényle; et $R_2$ et $R_3$ sont tels que définis dans la revendication 2 et $R_4$ est tel que défini dans la revendication 1.

5. Composés selon la revendication 4, dans lesquels $R_1$ représente un groupe cyclopropyle ou un groupe -$C(CH_3)=CH_2$; $R_4$ représente un atome d'hydrogène; et $R_2$ et $R_3$ sont tels que définis dans la revendication 2.

6. Composés selon l'une quelconque des revendications 1 à 5, choisis parmi:
le N-(5-benzofuryl)-2-cyano-3-cyclopropyl-3hydroxy-prop-2-énamide;
le N-[2-(8-chlorodibenzofuryl)]-2-cyano-3-cyclopropyl-3-hydroxy-prop-2-énamide;
le N-[2-(8-fluorodibenzofuryl)]-2-cyano-3-cyclopropyl-3-hydroxy-prop-2-énamide;
et leurs sels d'addition avec les bases.

7. Procédé de préparation de composés selon l'une quelconque des revendications 1 à 6, caractérisé en ce que l'on fait réagir
a) un composé de formule (II):

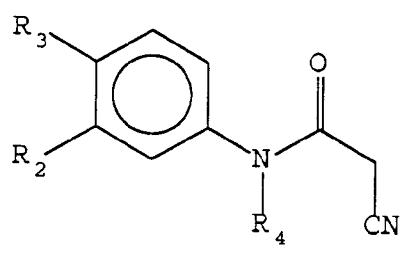

(II)

(dans laquelle $R_2$, $R_3$ et $R_4$ sont tels que définis dans la revendication 1) avec une base forte (le cas échéant, en présence d'un catalyseur), puis fait réagir le produit avec un composé de formule (III):

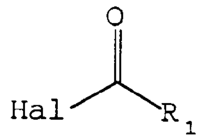

(III)

(dans laquelle Hal représente un atome d'hydrogène et $R_1$ est tel que défini dans la revendication 1); ou

(b) un composé de formule (II), telle que définie ci-dessus, avec une base forte (le cas échéant, en présence d'un catalyseur), puis fait réagir le produit avec un composé de formule ($III_A$):

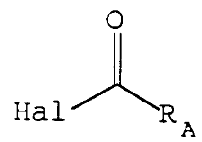

($III_A$)

(dans laquelle Hal représente un atome d'halogène et $R_A$ représente le groupe $R_1$ tel que défini ci-dessus, portant, en outre, un groupe protecteur), pour donner un composé de formule ($I_A$):

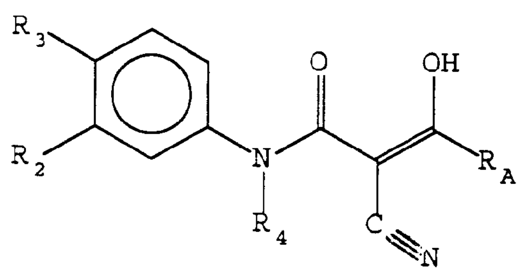

($I_A$)

(dans laquelle $R_A$, $R_2$, $R_3$ et $R_4$ sont tels que définis ci-dessus), puis élimine le groupe protecteur, pour obtenir le composé de formule ($I_A$) correspondant, dans lequel $R_A$ représente un groupe $R_1$ tel que défini dans la revendication 1.

**8.** Procédé selon la revendication 7, dans lequel la base forte est l'hydrure de sodium.

**9.** Procédé selon la revendication 7 ou la revendication 8, dans lequel, dans le composé de formule ($III_A$), $R_A$ représente un groupe protecteur arylséléno.

**10.** Procédé selon l'une quelconque des revendications 7 à 9, dans lequel, dans le composé de formule ($III_A$), $R_A$ représente un groupe protecteur phénylséléno.

**11.** Composés de formule (II) tels que définis dans la revendication 7.

**12.** Procédé selon l'une quelconque des revendications 7 à 10, dans lequel, le composé de formule (II) est préparé par réaction d'un composé de formule (IV)

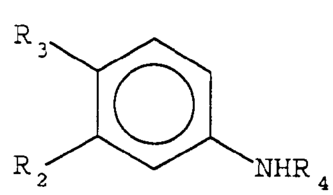

(IV)

(dans laquelle $R_2$, $R_3$ et $R_4$ sont tels que définis dans la revendication 7) avec de l'acide cyanoacétique.

**13.** Procédé selon la revendication 12, dans lequel la réaction entre le composé de formule (IV) et l'acide cyanoacétique, est réalisée en présence de dicyclohexylcarbodiimide ou de pentachlorure de phosphore, et en présence d'un tétrahydrofurane ou d'un dichlorométhane anhydre.

**14.** Compositions pharmaceutiques comprenant, comme substance active, au moins un composé de formule (I) ou ses tautomères, selon l'une quelconque des revendications 1 à 6, ou un des ses sels d'addition avec les bases pharmacologiquement acceptables, en association avec un ou plusieurs diluants, véhicules et/ou excipients pharmacologiquement acceptables.

**15.** Composés de formule (I) ou leurs tautomères, tels que définis dans l'une quelconque des revendications 1 à 6, et leurs sels d'addition avec les bases pharmacologiquement acceptables, pour leur utilisation en thérapeutique.

**16.** Utilisation d'un composé de formule (I), ou de son tautomère, selon l'une quelconque des revendications 1 à 6, et de ses sels d'addition avec les bases pharmacologiquement acceptables, dans la préparation d'un médicament servant à traiter la polyarthrite rhumatoïde, les maladies inflammatoires chroniques d'origine immunitaire ou non, le cancer, le diabète, la prévention du rejet aigu ou chronique suivant les transplantations d'organes et la resténose post-angioplastie.

Office européen des brevets

# RAPPORT DE RECHERCHE EUROPEENNE

Numero de la demande
EP 94 40 2478

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X<br><br><br><br>A | DE-A-25 55 789 (HOECHST AG) 7 Juillet 1977<br>* revendications 1,2; exemples *<br>* page 44, alinéa 3 - page 45, ligne 2 * | 1-6,11, 14-16<br><br><br>7-10,12, 13 | C07D307/91<br>C07D307/79<br>C07C255/23<br>A61K31/275<br>A61K31/34 |
| X | FR-A-2 313 031 (HOECHST AG) 31 Décembre 1976<br>* revendications; exemple 23; tableau C * | 1-6, 14-16 | |
| X | WO-A-91 17748 (HOECHST AG) 28 Novembre 1991<br>* page 1, ligne 1 - page 6, ligne 17; exemples 62,71,81,82 * | 1-6, 14-16 | |
| A | EP-A-0 551 230 (ROUSSEL-UCLAF) 14 Juillet 1993<br>* le document en entier * | 1-16 | |
| | | | **DOMAINES TECHNIQUES RECHERCHES (Int.Cl.6)** |
| | | | C07D<br>C07C |

Le présent rapport a été établi pour toutes les revendications

| Lieu de la recherche | Date d'achèvement de la recherche | Examinateur |
|---|---|---|
| LA HAYE | 18 Janvier 1995 | Paisdor, B |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)